# EUROPEAN PATENT APPLICATION

(11) **EP 3 031 393 A1**
(43) Date of publication of application: **15.06.2016**
(21) Application number: 15195424.5
(22) Date of filing: 19.11.2015
(51) Int. Cl.: A61B 5/024, A61B 5/11, A61B 5/00, G04G 21/02, G04B 47/06

(54) **WRIST DEVICE FOR OBSERVING PHYSIOLOGICAL MEASUREMENT DATA**

(30) Priority: 09.12.2014 US 201414564899
(71) Applicant: Polar Electro Oy, 90440 Kempele (FI)
(72) Inventor: Kampman, Ville, 90440 Kempele (FI); Komulainen, Olli, 90440 Kempele (FI)
(74) Representative: Kolster Oy Ab

(57) **Abstract**

There is provided a wrist device comprising: a first scale and a first hand, an actuator member configured to control movement of the first hand, at least one processor and at least one memory including a computer program code, wherein the at least one memory and the computer program code are configured, with the at least one processor, to cause the wrist device at least to: obtain physiological measurement data of a user, and cause, based on the physiological measurement data, the actuator member to move the first hand to indicate a physiological value on the first scale.

## Description

### FIELD OF THE INVENTION

The invention relates to a wrist device for observing physiological measurement data.

### BACKGROUND OF THE INVENTION

Wrist devices may be used to observe user's physiological information, such as heart rate for example. Usability of the wrist device may be affected on how the physiological information is indicated to the user. Thus, solutions enhancing the indication may be beneficial for the usability and attractiveness of the wrist device.

### BRIEF DESCRIPTION OF THE INVENTION

According to an aspect, there is provided the subject matter of the independent claim. Some embodiments are defined in the dependent claims.

According to an aspect, there is provided a wrist device comprising: a first scale and a first hand; an actuator member configured to control movement of the first hand; at least one processor and at least one memory including a computer program code, wherein the at least one memory and the computer program code are configured, with the at least one processor, to cause the wrist device at least to: obtain physiological measurement data of a user; and cause, based on the physiological measurement data, the actuator member to move the first hand to indicate a physiological value on the first scale.

In an embodiment, the physiological measurement data comprises physical activity information and wherein the at least one memory and the computer program code are configured, with the at least one processor, to cause the wrist device further to: cause, based on the physical activity information, the actuator member to move the first hand to indicate physical activity of the user.

In an embodiment, the at least one memory and the computer program code are configured, with the at least one processor, to cause the wrist device further to: process the physical activity information into accumulated physical activity information; cause, based on the accumulated physical activity information, the actuator member to move the first hand to indicate accumulated physical activity of the user.

In an embodiment, the physical activity information comprises motion activity information, and wherein the at least one memory and the computer program code are configured, with the at least one processor, to cause the wrist device further to: cause, based on the motion activity information, the actuator member to move the first hand to indicate at least one of the following: motion activity of the user in minutes and motion activity of the user in percent of a daily target motion activity.

In an embodiment, the physiological measurement data comprises weight information, and wherein the at least one memory and the computer program code are configured, with the at least one processor, to cause the wrist device further to: cause, based on the weight information, the actuator member to move the first hand to indicate weight of the user.

In an embodiment, the wrist device further comprises: an indication mode selector configured to enable selecting an indication mode of the wrist device, wherein the indication mode is selectable from at least one of the following: a physical activity indication, a weight indication, a time indication and the indication of the elapsed time, and wherein the at least one memory and the computer program code are configured, with the at least one processor, to cause the wrist device further to: obtain selected indication mode from the indication mode selector; and cause the actuator member to move the first hand according to the selected indication mode.

In an embodiment, the indication mode selector comprises a rotatable mechanical selector.

In an embodiment, the wrist device further comprises: an indication mode indicator comprising a mode indicator hand, wherein the at least one memory and the computer program code are configured, with the at least one processor, to cause the wrist device further to: cause the mode indicator to indicate the selected indication mode.

In an embodiment, the wrist device further comprises: a heart rate zone indicator comprising a heart rate zone indicator hand, wherein the at least one memory and the computer program code are configured, with the at least one processor, to cause the wrist device further to: cause, based on the physical activity information, the heart rate zone indicator to indicate heart rate zone of the user.

In an embodiment, the at least one memory and the computer program code are configured, with the at least one processor, to cause the wrist device further to: obtain selection of at least one of the following indication modes: the physical activity indication, the weight indication, the time indication and the indication of the elapsed time; and cause the heart rate zone indicator to indicate heart rate zone of the user.

In an embodiment, the first scale comprises values on a circle, and wherein zero point of the first scale is situated at a bottom of the circle.

In an embodiment, the first scale comprises heart rate values on the circle, wherein heart rate values represent at least one heart rate zone, and wherein the maximum heart rate value is situated at the bottom of the circle.

One or more examples of implementations are set forth in more detail in the accompanying drawings and the description below. Other features will be apparent from the description and drawings, and from the claims.

### BRIEF DESCRIPTION OF THE DRAWINGS

Embodiments of the present invention are described below, by way of example only, with reference to the accompanying drawings, in which
Figure 1 illustrates a wrist device according to an embodiment of the invention;
Figure 2 illustrates an embodiment of the invention;
Figures 3A to 3E illustrate some embodiments of the invention;
Figure 4 illustrates physiological information acquiring according to an embodiment of the invention;
Figure 5 illustrates a block diagram of the wrist device according to an embodiment of the invention; and
Figure 6 illustrates an embodiment of the invention.

### DETAILED DESCRIPTION OF THE INVENTION

The following embodiments are exemplary. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

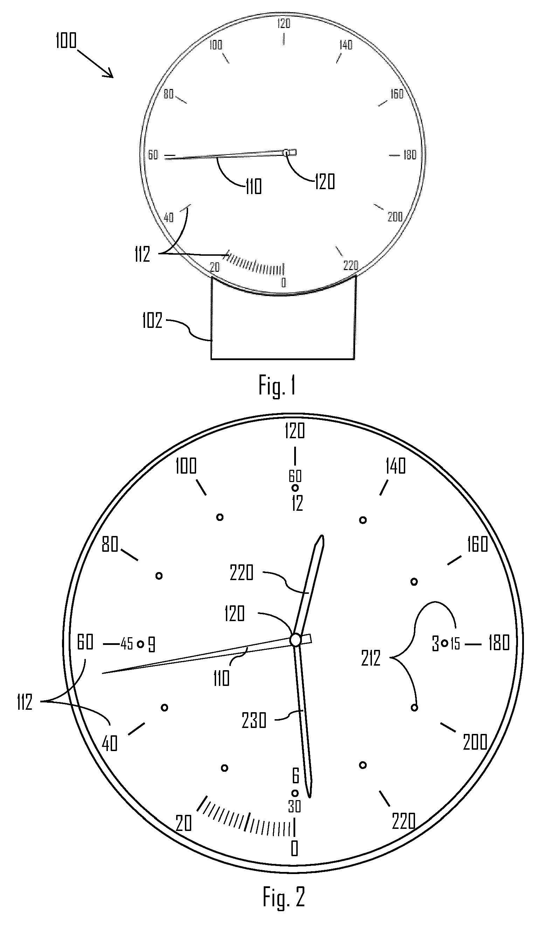

Figure 1 illustrates a wrist device according to an embodiment of the invention. Referring to Figure 1, the wrist device 100 comprises a first scale 112 and a first hand 110. The first scale 112 is illustrated in Figure 1 with a simplified form. Thus, the first scale 112 may comprise more line(s) and/or number(s).

The wrist device 100 further comprises an actuator member 120 configured to control movement of the first hand 110. The actuator member 120 may be able to rotate the first hand 110 in order to point a certain value on the first scale 112.

Further, the wrist device 100 comprises at least one processor and at least one memory including a computer program code, wherein the at least one memory and the computer program code are configured, with the at least one processor, to cause the wrist device 100 at least to: obtain physiological measurement data of a user, and cause, based on the physiological measurement data, the actuator member 120 to move the first hand 110 to indicate a physiological value on the first scale 112. Thus, the wrist device 100 may, for example, obtain a heart rate measurement, and indicate the heart rate according to the said measurement.

In an embodiment, the wrist device 100 processes the obtained physiological measurement data into a physiological metric characterizing the physiological behavior of the user. Therefore, the physiological metric may be indicated using the actuator member 120, the first hand 110 and the first scale 112.

The wrist device 100 may enhance the attractiveness and usability of observing the physiological measurement data, such as physical activity information. The first hand 110 may be easier to observe during a physical exercise, as it may immediately indicate the value and/or value area of the observed physiological value. Thus, the physiological value may be observed by giving a glance, by the user, to the wrist device 100. This may mean that the user has more time to concentrate on the present task at hand, such as the physical exercise.

The wrist device 100 may further comprise a casing, wherein the casing may give at least part of the wrist device 100 a form. The casing may enclose the at least one processor and the at least one memory. The wrist device 100 may comprise a wrist strap 102 for detachably fixing the wrist device 100 around the user's wrist. The wrist device 100 may also comprise a cover, such as a cover glass and/or cover plastic, on top of the first scale 112 and the first hand 110. The cover may be substantially transparent and may be used to protect the wrist device 100.

In an embodiment, the first hand 110 and the first scale 112 are analog display members. Thus, the first scale 112 may be printed on a background comprised in the wrist device 100. Similarly, the first hand 110 may be a mechanical pointer rotated by the actuator member 120. The word analog may mean that the display members are not digital.

In an embodiment, the background is static. Therefore, the values indicated by the first scale 112 may not be changed.

In an embodiment, the actuator member 120 comprises an electronic motor. The electronic motor may receive instructions from the wrist device 100, and more particularly from the processor to move and/or rotate the first hand 110 according to the instructions. The instructions may be based on the physiological measurement data, as described earlier.

Figure 2 illustrates an embodiment of the invention. Referring to Figure 2, the wrist device 100 may comprise a second scale 212, a second hand 220 and a third hand 230, wherein the second scale 212, the second hand 220 and the third hand 230 may be configured together to indicate time. Thus, the wrist device 100 may be used to indicate time together with indicating the physiological value.

In an embodiment, the first hand 110 is further configured to indicate time together with the second and third hands 220, 230 on the second scale 212. The first hand 110 may indicate seconds on the second scale 212.

The second scale 212 may be comprised in the same background as the first scale 112. Thus, the second scale 212 may be, for example, printed on the background. The second scale 212 may comprise hours ranging from 1 to 12, and minutes and/or seconds ranging from 1 to 60. The second hand 220 may indicate hours and the third hand 230 may indicate minutes, for example.

In an embodiment, the hours range from 1 to 24 hours.

In an embodiment, the second scale 212 and the second and third hands 220, 230 are analog display members.

In an embodiment, the wrist device 100 does not comprise digital display elements. Thus, the first and second scales 112, 212 and the hands 110, 220, 230 may all be analog display elements. Similarly, if other display elements are used, as described later, they all may be analog display elements.

The second and third hands 220, 230 may be moved and/or rotated by the actuator member 120. The actuator member 120 may comprise different parts which produce the moving energy to the first hand 110 and to the second and third hands 220, 230 respectfully. Thus, the rotation of the hands 110, 220, 230 may be independent.

In an embodiment, the first hand 110 is colored red, and the second and the third hands 220, 230 are colored with some other color than red.

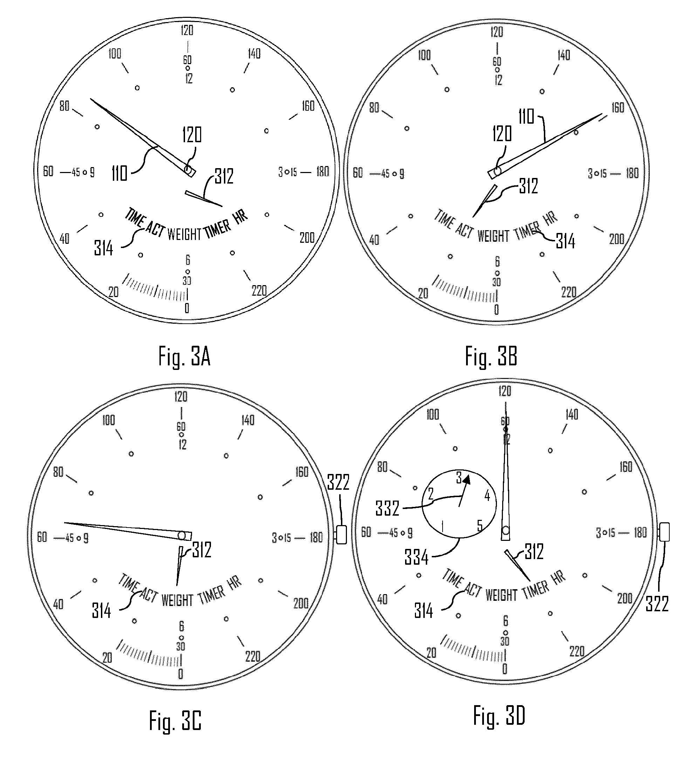

Let us now look closer on indication by the wrist device 100 looking at the embodiments of Figures 3A to 3E. Referring to Figures 3A to 3E, the wrist device 100 may be configured to display different things, such as different physiological values. The physiological measurement data, obtained by the wrist device 100, may comprise physical activity information, such as heart rate information and/or motion activity information, and/or weight information. The wrist device 100 may use the said information to indicate physical activity, such as heart rate and/or motion activity, of the user and/or weight of the user. More particularly, the at least one memory and the computer program code may be configured, with the at least one processor, to cause the wrist device 100 to cause the actuator member 120 to move the first hand 110 to indicate the wanted value based on the said information.

Referring to Figure 3A, the wrist device 100 may comprise an indication mode indicator 312, 314. The indication mode indicator 312, 314 may be configured to indicate an indication mode of the wrist device 100. The indication modes may be, for example, a physical activity indication, wherein the user's physical activity, such as the heart rate and/or motion activity, is indicated, a weight indication, wherein the user's weight is indicated, a time indication, wherein the current time is indicated and/or an elapsed time indication, wherein the elapsed time of a time measurement is indicated.

In an embodiment, the wrist device 100 processes the physical activity information into accumulated physical activity information, and causes, based on the accumulated physical activity information, the actuator member 120 to move the first hand 110 to indicate accumulated physical activity of the user. This way the wrist device 100 may, for example, indicate the time spent active and/or inactive by the user. The accumulated physical activity may comprise, for example, accumulated activity for a period of one day.

The wrist device 100 may cause the indication mode indicator 312, 314 to indicate current indication mode, such as selected indication mode. The current indication mode may be selected, for example, by the user as described later. Further, the wrist device 100 may select, for example, physical activity indication if it determines that the user is performing a physical activity. The determination may be based on obtaining and/or receiving physical activity information. Similarly, the selected indication mode may be weight indication when the wrist device 100 obtains weight information.

In an embodiment, the wrist device 100 selects the indication mode on a round-robin cycle. Therefore, at least some of the indication modes are selected and displayed on a loop. For example, the wrist device 100 may determine that the user is performing a physical activity, and displays heart rate and motion activity in turns for a predetermined time.

Still referring to Figure 3A, the indication mode indicator 312, 314 may comprise a mode indicator hand 312 configured to indicate the selected indication mode 314. In the example of Figure 3A, the mode indicator hand 312 may indicate that the selected indication mode may be the physical activity indication, and more precisely a heart rate indication. Thus, the actuator member 120 may move the first hand 110 so that it points to a value on the first scale 112, wherein the value may be a physical activity value, such as a heart rate value. In the example of Figure 3A, the heart rate may be determined to be circa 85 beats per minute (bpm).

In an embodiment, the mode indicator hand 312 is moved by a second actuator member. The indication modes 314 may be printed on the same background as the scales 112, 212, for example. Thus, the mode indication may also be analog.

Referring to Figure 3B, the mode indicator hand 312 may indicate that the selected indication mode may be the physical activity indication, and more precisely a motion activity indication. The indication may be based on the motion activity information comprised in the physical activity information. The wrist device 100 may cause the actuator member 120 to move the first hand 110 to indicate accumulated motion activity of the user, for example. Therefore, the first hand 110 may indicate a motion activity value on the first scale 112. In the example, of Figure 3B, the motion activity value may be circa 160 minutes. Thus, the user may have been active for around two and a half hours during the day.

In an embodiment, the first hand 110 is configured to indicate the motion activity in minutes on the second scale 212. Thus, in the example of Figure 3B, the first hand 110 may indicate that the user may have been active for circa 10 minutes.

In an embodiment, the second scale 212 comprises indicators for motion activity. This may mean that the second scale 212 comprises hours, minutes and the indicators for motion activity. The indicators may be used to indicate, by pointing the first hand 110, motion activity of the user in minutes. For example, one hour in the second scale 212 may correspond to 30 motion activity minutes. Therefore, in the example of Figure 3B, the first hand 110 may indicate that the user has been active for circa 60 minutes.

In an embodiment, the wrist device 100 causes, based on the motion activity information, the actuator member 120 to move the first hand 110 to indicate motion activity of the user in minutes and/or motion activity of the user in percent of a daily target motion activity. The physical activity may be indicated similarly. Further, the accumulated motion activity may be indicated as described above. The daily target motion activity may be a user specific and/or configurable value.

In an embodiment, the motion activity is defined by a predetermined threshold, wherein the motion activity calculation starts when the motion activity is over the predetermined threshold.

In an embodiment, the daily target motion activity is 30 minutes.

In an embodiment, the daily target motion activity is 60 minutes.

In an embodiment, the daily target motion activity is 120 minutes.

Referring to Figure 3C, the mode indicator hand 312 may indicate that the selected indication mode may be the weight indication. The wrist device 100 may cause the actuator member 120 to move the first hand 110 to indicate weight of the user. In the example of Figure 3C, the first hand 110 may indicate that the weight is circa 65 kilograms. Naturally, the weight may be indicated in pounds depending on the country configuration of the wrist device 100.

In an embodiment, the weight indication depends on the country and/or region configuration. For example, if the user selects United States of America as his and/ hers country, the wrist device 100 may indicate the weight in pounds. In another example, the user may select Finland as his and/or hers country, and thus the wrist device 100 may indicate the weight in kilograms.

Still referring to Figure 3C, the wrist device 100 may comprise an indication mode selector 322 configured to enable selecting the indication mode of the wrist device 100, wherein the indication mode may be selectable from at least one of the following: the physical activity indication, such as the heart rate indication and/or the motion activity indication, the weight indication, the time indication and the indication of elapsed time.

The wrist device 100 may obtain the selected indication mode from the indication mode selector 322, and cause the actuator member 120 to move the first hand 110 according to the selected indication mode. Thus, the indication may be done using at least one of the first and the second scales 112, 212. For example, the user selects the heart rate indication using the indication mode selector 322. The indication mode indicator 312, 314 may then indicate the heart rate indication, and the actuator member 120 may move the first hand 110 on the first scale 112 according to the current heart rate of the user.

In an embodiment, the indication mode selector 322 comprises and/or is a rotatable mechanical selector. The user may rotate the mechanical selector for a certain amount, and the wrist device 100 may detect the rotation and change the indication mode according to the amount of the rotation. For example, if there are five different indication modes, circa 72 degrees of rotation may cause the wrist device 100 to change the indication mode to the next indication mode.

Referring to Figure 3D, the selected indication mode may be the indication of elapsed time, wherein the elapsed time of the time measurement may be indicated. The wrist device 100 may obtain an input to start the time measurement, and as a response to the obtaining, cause an entity, comprising the second scale 212, the second hand 220 and the third hand 230, to indicate elapsed time of the time measurement. This may mean that the wrist device 100 causes the actuator member 120 to actuate the second and third hands 220, 230 on the second scale 212 according to the elapsed time. The second and the third hands 220, 230 may indicate seconds and minutes according to the time measurement. Further, when the time measurement is started, the wrist device 100 may cause the actuator member 120 to move the first hand 110 to indicate the physical activity, such as heart rate and/or motion activity, of the user on the first scale 112.

In an embodiment, the first hand 110 is used by the wrist device 100 to indicate seconds on the second scale 212 according to the time measurement. In such case, the second and the third hands 220, 230 may be used to indicate minutes and hours.

The wrist device 100 may comprise a heart rate zone indicator 332, 334. The wrist device 100 may cause, based on the heart rate information, the heart rate zone indicator 332, 334 to indicate heart rate zone of the user. This may mean that the heart rate zone indicator 332, 334 indicates the current heart rate zone of the user according to the heart rate information. For example, if the user's heart rate at a certain time is 150 bpm, the wrist device 100 may determine in which heart rate zone the 150 bpm belongs to and cause the heart rate zone indicator 332, 334 to indicate the determined heart rate zone.

The heart rate zones may comprise, for example, five heart rate zones, each zone representing a certain group of heart rate values. For example, the heart rate zone 5 may correspond to heart rate values of a user from 90 % to 100 % from maximum heart rate. Thus, these values may be, for example, 171 bpm to 190 bpm for a certain user. The maximum heart rate value may be estimated roughly to be 220 bpm - age of the user.

In an embodiment, the heart rate zone 1 corresponds to heart rate values ranging from 50 % to 60 % of the maximum heart rate, the heart rate zone 2 corresponds to heart rate values ranging from 60 % to 70 % of the maximum heart rate, the heart rate zone 3 corresponds to heart rate values ranging from 70 % to 80 % of the maximum heart rate, the heart rate zone 4 corresponds to heart rate values ranging from 80 % to 90 % of the maximum heart rate and the heart rate zone 5 corresponds to heart rate values ranging from 90 % to 100 % of the maximum heart rate. However, the wrist device 100 may be used to change the limits for each of the heart rate zones by the user.

In an embodiment, the heart rate zone indicator 332, 334 comprises a heart rate zone indicator hand 332 configured to indicate the heart rate zone 334 of the user.

In an embodiment, the heart rate zone indicator hand 332 is moved by a third actuator member controlled by the wrist device 100. The heart rate zones, ranging from 1 to 5, may be printed on the same background as the scales 112, 212, for example. Thus, the heart rate zone indication may also be analog.

In an embodiment, the heart rate zone indicator 332, 334 is used to indicate the heart rate zone of the user even if the selected indication mode would be different from the physical activity indication mode, and more precisely the heart rate indication mode. Thus, the wrist device 100 may indicate the heart rate zone using the heart rate zone indicator 332, 334, and at the same time use, for example, the first hand 110 to indicate elapsed time of a time measurement, the time, the motion activity and/or the weight.

In an embodiment, the wrist device 100 obtains selection of at least one of the following indication modes: the physical activity indication, the weight indication, the time indication and the indication of the elapsed time, and causes the heart rate zone indicator 332, 334 to indicate heart rate zone of the user.

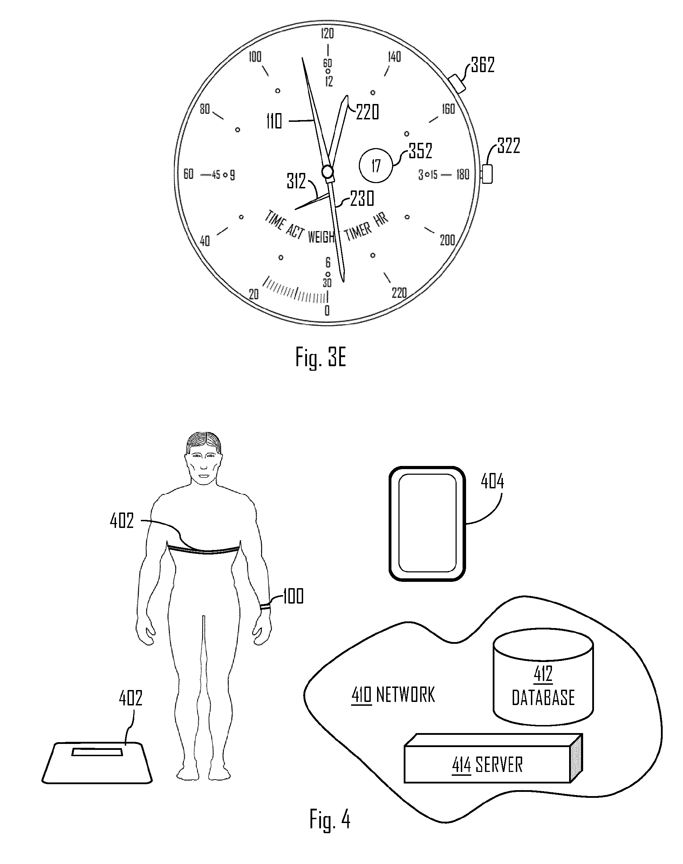

Referring to Figure 3E, selected indication mode may be the time indication, wherein the current time may be indicated. This may mean that the wrist device 100 uses at least one of the first hand 110, the second hand 220 and the third hand 230 to indicate current time on the second scale 212. In such case, the first hand 110 may be used to indicate seconds.

In an embodiment, the wrist device 100 uses at least one of the first hand 110, the second hand 220 and the third hand 230 to indicate elapsed time of the time measurement. Therefore, changing the indication mode, for example, from the mode selector 322 may change the functionality of at least some of the hands 110, 210, 230.

The wrist device 100 may further comprise an indicator 352. The indicator 352 may be used to indicate the current date and/or day. In the example of Figure 3E, the indicator 352 may indicate that the current date is 17^{th}.

In an embodiment, the indicator 352 is used to indicate connectivity status of the wrist device 100. As later described in more detail, the wrist device 100 may comprise a wireless connection circuitry. The indicator 352 may be used to indicate the status of the wireless connection, such as connected, not connected and/or searching, wherein the connected may mean that the wrist device 100 is connected to another device, the not connected may mean that the wrist device 100 is not connected to another device and the searching may mean that the wrist device 100 is searching for another device to connect to. The indication by the indicator 352 may be analog.

In an embodiment, the wrist device 100 obtains an input requesting to start or stop the physical activity measurement, and sends a control message to at least one physical activity sensor providing the physical activity information by performing the physical activity measurement, wherein the control message causes the at least one physical activity sensor to start or stop the physical activity measurement. The at least one physical activity sensor may comprise heart activity sensor(s) and/or motion activity sensor(s). This is discussed later in more detail.

Still referring to Figure 3E, the wrist device 100 may comprise a mechanical button 362, wherein the mechanical button 362 may be configured to enable starting and stopping of at least one of the following: the time measurement and the physical activity measurement. For example, when the wrist device 100 is in the elapsed time indication mode, by pressing the mechanical button 362 the wrist device 100 may start to measure elapsed time and indicate it to the user. By pressing the mechanical button 362 again, the time measurement may be stopped and/or paused. The time measurement may be continued by pressing the mechanical button 362 again and/or set to zero by pressing it longer. Similarly, the mechanical button 362 may be used to start and stop the physical activity measurement.

In an embodiment, by pressing the mechanical button 362, the physical activity measurement is displayed together with the time measurement. Thus, for example, the time measurement may be displayed together with the heart rate by the wrist device 100.

In an embodiment, the wrist device 100 determines that the mechanical button 362 is pressed for a predetermined time, and as a response starts to search for external sensor device(s). If a suitable sensor device is found, it may be paired with the wrist device 100.

Figure 4 illustrates physiological information acquiring according to an embodiment of the invention. Referring to Figure 4, the wrist device 100 may be used together with other device(s), such as external sensor device(s) 402, a portable electronic device 404 and a server 414 residing in a network 410. The wrist device 100 may be connected to the external sensor device(s) 402, to the portable electronic device 404 and to the server 414 by a communication link that may enable transfer of data. Therefore, the physiological information, such as the physiological measurement data, may be transferred between the wrist device 100 and the external sensor device(s) 402. For example, the external sensor device(s) 402 may transmit the physiological information to the wrist device 100 and the wrist device 100 may receive the transmitted physiological information. Thus, the wrist device 100 may receive, from the at least one external sensor device 402, at least one of the following: the physical activity information, such as the heart rate information and/or the motion activity information, and the weight information.

Similarly, data may be transferred between the wrist device 100, the portable electronic device 404 and the server 414. Thus, it may be possible to, for example, transmit, by the wrist device 100, physical activity file(s), characterizing the physical activity of the user, to the portable electronic device 404 and/or to the server 414. The portable electronic device 404 and/or the server 414 may receive the transmitted file(s) and make the file(s) available for viewing on a bigger screen and/or with greater detail, for example. The physical activity file(s) may be at least partly based on the physiological information.

The wrist device 100, the portable electronic device 404 and the server 414 may comprise a memory to which the physiological information may be stored. Further, the network 410 may comprise a database 412, such as a training database, to be used to store physiological information and/or physical activity file(s). The wrist device 100 may, for example, transmit physiological information and/or physical activity file(s) to the server 414 that causes the server 414 to store the transmitted data to the database 412.

In an embodiment, the wrist device 100 transmits, to the database 412, at least one of the following: the physical activity information, such as the heart rate information and/or the motion activity information, the weight information and the elapsed time of the time measurement.

Still referring to Figure 4, the external sensor device(s) 402 may comprise sensors, such as a heart rate transmitter, a stride sensor, a positioning sensor, a cadence sensor, a power sensor and a weighing scale, to mention a few. The heart rate transmitter may comprise at least one electrical, optical and/or bioimpedance sensor to measure user's heart activity, such as heart rate. The positioning sensor may comprise a GPS, a magnetometer and/or a Bluetooth sensor. Thus, the positioning may be based on, for example, GPS location and/or Bluetooth location. The magnetometer may provide direction data based on magnetic fields on earth and/or inside structures. The weighing scale may be used to determine user's weight and thus produce the weight information.

The optical heart activity sensor may detect the user's heart activity by optical heart rate measurement, which may comprise sending a light beam towards user's skin and measuring the bounced and/or emitted light from the user's skin. The light beam may alter when travelling through the user's veins and the alterations may be detected by the optical heart rate activity sensor. By using the detected data, heart rate of the user may be determined by the wrist device 100 and/or the heart rate transmitter.

The bioimpedance heart activity sensor may detect user's heart activity by bioimpedance measurement. The bioimpedance measurement may be based on transmitting a radio signal into user's skin, and observing changes in the radio signal due to impedance changes caused by, for example, blood volume changes. Thus, the user's heart activity, such as heart rate, may be determined by the wrist device 100 and/or the heart rate transmitter from the data produced by the bioimpedance sensor.

In an embodiment, the electrical sensor(s) for heart activity measurement are based on Electrocardiography (EKG) measurement.

In an embodiment, the weighing scale further comprises a sensor configuration for determining user's percentage of fat. The percentage of fat may be a part of the weight information.

In an embodiment, the portable electronic device 404 is a mobile phone, a smart phone, a palm device, a tablet computer, phablet and/or a portable digital assistant.

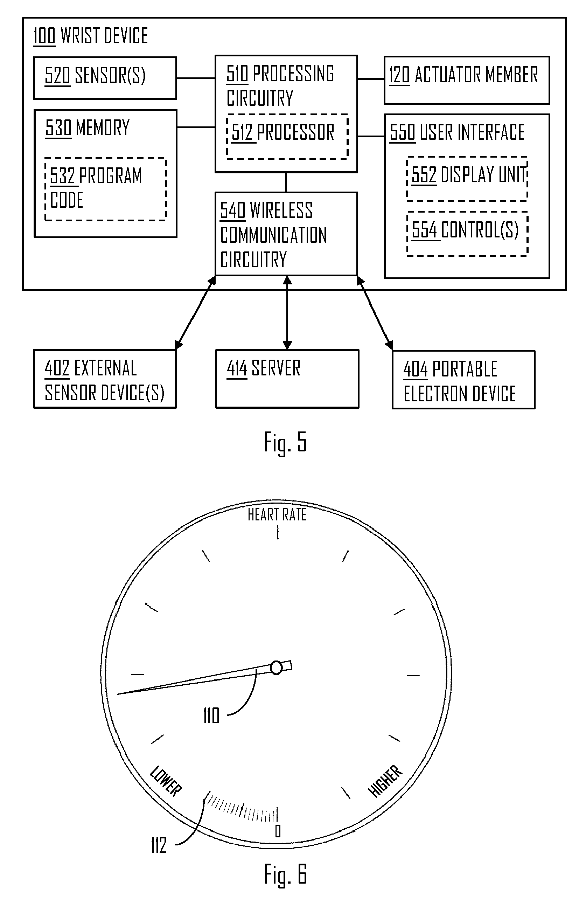

Figure 5 illustrates a block diagram of the wrist device 100 according to an embodiment of the invention. Referring to Figure 5, the wrist device 100 may comprise at least one processor 512. In an embodiment, the at least one processor 512 is comprised in a processing circuitry 510. The wrist device 100 may further comprise at least one memory 530, wherein the at least one memory 530 may comprise a computer program code 532. The at least one memory 530 and the computer program code 532 may be configured, with the at least one processor 512, to perform the above-mentioned functions of the wrist device 100.

In an embodiment, the processing circuitry 510 may be configured to perform the above-mentioned functions of the wrist device 100.

The wrist device 100 may further comprise wireless communication circuitry 540 configured to enable the wrist device 100 to transfer data between the wrist device 100 and at least one other electronic device, such as the external sensor device(s) 402, the portable electronic device 404 and the server 414. The wireless communication circuitry 540 may be based on Bluetooth® specifications, e.g. Bluetooth Low Energy, and/or Near-Field-Communication (NFC) technology, wherein the NFC technology may enable data transfer on short distances. However, the wireless communication circuitry 540 may not be limited to these technologies.

The wrist device 100 may comprise sensor(s) 520 configured to measure the physical activity information, such as the heart rate information and/or the motion activity information. The sensor(s) 520 may be enclosed by the casing of the wrist device 100.

The sensor(s) 520 may comprise at least one heart activity sensor, such as an optical heart activity sensor, such as a PPG (photoplethysmography) sensor, a bioimpedance sensor and/or an electrical heart activity sensor. Further, besides these types of heart activity sensors, also other types of biosignal measurement sensors may be embedded into the sensor(s) 520. These types include but are not limited to the following: a Laser Doppler-based blood flow sensor, a magnetic blood flow sensor, an Electromechanical Film (EMFi) pulse sensor, a polarization blood flow sensor.

The sensor(s) 520 may comprise a motion sensor configured to measure motion induced by the user to the wrist device 100 by moving hand in which the user wears the wrist device 100. The motion sensor may use other motion data, such as location data of the user, to determine user's motion. The motion sensor may measure user physical activity, and provide the physical activity information to the wrist device 100.

In an embodiment, the motion sensor(s) comprise at least one of the following: an accelerometer, a magnetometer, and a gyroscope.

In an embodiment, the motion sensor comprises an accelerometer and a gyroscope. The motion sensor may further comprise sensor fusion software for combining the accelerometer data and gyroscope data so as to provide physical quantities, such as acceleration data, velocity data, or limb trajectory data in a reference coordinate system having orientation defined by a predetermined gyroscope orientation.

In an embodiment, the motion sensor comprises a gyroscope and a magnetometer. The motion sensor may further comprise sensor fusion software to combine gyroscope data and magnetometer data so as to provide a reference coordinate system for the gyroscope based on the Earth magnetic field measured by the magnetometer. In general, the sensor fusion software described above may combine measurement data acquired from at least two motion sensors such that measurement data acquired from one motion sensor is used to establish the reference coordinate system for the measurement data acquired from at least one other motion sensor.

In an embodiment, the sensor(s) 520 further comprises at least one of the following sensors: a temperature sensor, a positioning sensor and a pressure sensor. The positioning sensor may utilize GPS and/or Bluetooth information for locating the user. Further, the positioning sensor may comprise a magnetometer. The position information produced by the positioning sensor may be comprised in the physical activity information, and more specifically in the motion activity information.

In an embodiment, the external sensor device(s) 402 are comprised in the wrist device 100. The external sensor device(s) 402 may be enclosed by the casing of the wrist device 100.

Still referring to Figure 5, the wrist device 100 may comprise the actuator member 120 configured to move the first hand 110 according to the instructions from the at least one processor 512. Further, the wrist device 100 may comprise the second and/or third actuator members that may also be controlled by the at least one processor 512.

The wrist device 100 may comprise user interface 550 configured to display and/or indicate the physiological information to the user and/or to allow interaction with the wrist device 100. The user interface 550 may comprise a display unit 552 that may comprise the hands 110, 220, 230, the scales 112, 212, the indication mode indicator 312, 314, the heart rate zone indicator 332, 334 and/or the indicator 352. The user interface 550 may comprise control(s) 554 that may comprise the indication mode selector 322 and/or the mechanical button 362.

In an embodiment, the display unit 552 comprises a digital component indicating at least some of the information in digital form. Thus, the physiological information, time and or date may be at least partly be displayed in digital form. This may be possible if the display unit 552 comprises, for example, digital display configured to display at least some of the said information.

In an embodiment, the first hand 110, the second hand 220 and/or the third hand 230 are illuminated. This may be achieved by using Light Emitting Diode(s) (LED), for example. Also phosphorus, or similar, may be used on the hands 110, 220, 230 to glow light. The LED(s) may be switched on and off from the controls 554. This may enhance the usability of the wrist device 100 in dark conditions.

Figure 6 illustrates an embodiment of the invention. Referring to Figure 6, the first scale 112 may comprise values on a circle, and wherein zero point of the first scale 112 may be situated at a bottom of the circle. Thus, the zero in the Figure 6 may illustrate the zero point. The bottom of the circle may comprise a certain section, for example, 10 to 20 % of the circles circumference, measured from the exact bottom point to opposing directions on the circumference. The first hand 110 may move clockwise on the circle.

In an embodiment, the first hand 110 moves counterclockwise on the circle.

In an embodiment, the first scale 112 comprises values from 0 to 240.

In an embodiment, the first scale 112 comprises heart rate values on the circle, wherein the heart rate values represent at least one heart rate zone, and wherein the maximum heart rate value is situated at the bottom of the circle. The at least one heart rate zone may comprise the previously mentioned heart rate zone(s) from 1 to 5. Further, the at least one heart rate zone may comprise all the user's heart rate values that may be possible to the user. Thus, the said heart rate zone may extend from user's resting heart rate value to the user's maximum heart rate value. The heart rate values may increase on the circle to clockwise direction, and as mentioned, the maximum heart rate value may be situated at the bottom of the circle. Therefore, 360 degrees on the circle may correspond to the user's heart rate values. Also, as was said previously, the bottom of the circle may comprise more than just the exact bottom point of the circle.

In an embodiment, the first scale 112 is arranged on an adaptive background. The background may be common to the other scale(s) and/or separate from the background(s) of the other scale(s). The adaptive background may be achieved by using digital display unit(s). The first scale 112 may be configured so that it represents heart rate values that are possible for the current user. For different users the first scale 112 may thus be different, as the possible heart rate values vary from user to user.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, described with regard thereto, and they are not limited to the precise configurations set forth in the given figures, as will be appreciated by one skilled in the art.

Embodiments, that may be performed by the processing circuitry 510 and/or the at least one processor 512, as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims. Therefore, all words and expressions should be interpreted broadly and they are intended to illustrate, not to restrict, the embodiment. It will be obvious to a person skilled in the art that, as technology advances, the inventive concept can be implemented in various ways. Further, it is clear to a person skilled in the art that the described embodiments may, but are not required to, be combined with other embodiments in various ways.

## Claims

1. A wrist device comprising:
a first scale and a first hand;
an actuator member configured to control movement of the first hand;
at least one processor and at least one memory including a computer program code, wherein the at least one memory and the computer program code are configured, with the at least one processor, to cause the wrist device at least to:
obtain physiological measurement data of a user; and
cause, based on the physiological measurement data, the actuator member to move the first hand to indicate a physiological value on the first scale.

2. The wrist device of claim 1, further comprising:
a second scale, a second hand and a third hand, wherein the second scale, the second hand and the third hand are configured together to indicate time.

3. The wrist device of any preceding claim, wherein the physiological measurement data comprises physical activity information and wherein the at least one memory and the computer program code are configured, with the at least one processor, to cause the wrist device further to:
cause, based on the physical activity information, the actuator member to move the first hand to indicate physical activity of the user.

4. The wrist device of claim 3, wherein the at least one memory and the computer program code are configured, with the at least one processor, to cause the wrist device further to:
process the physical activity information into accumulated physical activity information;
cause, based on the accumulated physical activity information, the actuator member to move the first hand to indicate accumulated physical activity of the user.

5. The wrist device of any preceding claim, wherein the physiological measurement data comprises weight information, and wherein the at least one memory and the computer program code are configured, with the at least one processor, to cause the wrist device further to: cause, based on the weight information, the actuator member to move the first hand to indicate weight of the user.

6. The wrist device of any preceding claim, wherein the at least one memory and the computer program code are configured, with the at least one processor, to cause the wrist device further to:
obtain input to start a time measurement;
cause an entity, comprising the second scale, the second hand and the third hand, to indicate elapsed time of the time measurement; and
cause the actuator member to move the first hand to indicate the physical activity of the user on the first scale.

7. The wrist device of any preceding claim, further comprising:
an indication mode selector configured to enable selecting an indication mode of the wrist device, wherein the indication mode is selectable from at least one of the following: a physical activity indication, a weight indication, a time indication and the indication of the elapsed time, and wherein the at least one memory and the computer program code are configured, with the at least one processor, to cause the wrist device further to:
obtain selected indication mode from the indication mode selector; and
cause the actuator member to move the first hand according to the selected indication mode.

8. The wrist device of any preceding claim, further comprising:
an indication mode indicator comprising a mode indicator hand, wherein the at least one memory and the computer program code are configured, with the at least one processor, to cause the wrist device further to:
cause the mode indicator to indicate the selected indication mode.

9. The wrist device of any preceding claim, further comprising:
a heart rate zone indicator comprising a heart rate zone indicator hand, wherein the at least one memory and the computer program code are configured, with the at least one processor, to cause the wrist device further to:
cause, based on the physical activity information, the heart rate zone indicator to indicate heart rate zone of the user.

10. The wrist device of any preceding claim, wherein the at least one memory and the computer program code are configured, with the at least one processor, to cause the wrist device further to:
obtain an input requesting to start or stop a physical activity measurement; and
send a control message to at least one physical activity sensor providing the physical activity information by performing the physical activity measurement, wherein the control message causes the at least one physical activity sensor to start or stop the physical activity measurement.

11. The wrist device of any of claims 6 to 10, further comprising:
a mechanical button, wherein the mechanical button is configured to enable starting and stopping of at least one of the following: the time measurement and the physical activity measurement.

12. The wrist device of any preceding claim, further comprising:
a wireless communication circuitry configured to enable the wrist device to transfer data between the wrist device and at least one other electronic device.

13. The wrist device of claim 12, wherein the at least one memory and the computer program code are configured, with the at least one processor, to cause the wrist device further to:
receive, from at least one external sensor device, at least one of the following: the physical activity information and the weight information.

14. The wrist device of any of claims 12 to 13, wherein the at least one memory and the computer program code are configured, with the at least one processor, to cause the wrist device further to:
transmit, to a training database, at least one of the following: the physical activity information, the weight information and the elapsed time of the time measurement.

15. The wrist device of any preceding claim, further comprising:
at least one sensor configured to measure the physical activity information; and
a casing enclosing the at least one processor, the at least one memory and the at least one sensor.

16. The wrist device of any preceding claim, wherein the first scale comprises values on a circle, and wherein zero point of the first scale is situated at a bottom of the circle.
